# EUROPEAN PATENT APPLICATION

(11) **EP 1 354 941 A1**
(43) Date of publication of application: **22.10.2003**
(21) Application number: 02447069.2
(22) Date of filing: 19.04.2002
(51) Int. Cl.: C12M 3/06, C12M 1/12

(54) **Apparatus and method for a cell culture in a bioreactor at high cell concentration**

(71) Applicant: Computer Cell Culture Center S.A., 7180 Seneffe (BE)
(72) Inventor: Miller, Alain, 7000 Mons (BE); Gatot, Christophe, 5032 Bossière (BE)
(74) Representative: Van Malderen, Joelle

(57) **Abstract**

The present invention is related to an apparatus and a method for the production of biological products of interest from a cell culture in a sonoperfused bioreactor (2) at high cell density, wherein a set of two pumps (5,7') and a set of two valves (8,9) working alternately are used, and a discontinuous recirculation of the cells in the bioreactor is ensured.

## Description

### Field of the invention

The present invention is related to a method and an apparatus for obtaining biological products from cells grown in a bioreactor at high cell concentration.

In particular, the present invention is related to a method and an apparatus including an efficient recirculation in the bioreactor of the cells contained in the spent growth medium.

### State of the art

Bioreactors are processing units used for the production of biological products of interest issued from prokaryotic or eukaryotic cells growing in said units. Said biological products of interest are, for example, proteins (possibly recombinant proteins), peptides, nucleic acids or aminoacids, which may then serve for the synthesis of specific biotech products like active pharmaceutical compounds (vaccines, monoclonal antibodies) or industrial compounds (enzymes).

These bioreactors may work at low cell concentrations. However, the culture at high cell concentrations of prokaryotic and eukaryotic cells (whether anchorage-dependent or not), insect or plant cells at high cell concentrations, presents a series of advantages such as reduced fixed costs (bioreactor of small size, reduced validated space and head count), improved quality of the biological products obtained and increased productivity (Miller, 2001).

Classically, the production of biological compounds using a bioreactor comprises the steps of growing said cells in an appropriate growth medium, recovering said biological compounds from the cell growth medium followed by purification.

Methods have already been developed in order to increase cell density and volumetric productivity in bioreactors. Perfusion is one of them. Perfusion involves continuous addition of fresh medium and removal of spent medium containing the searched biological compound.

Perfusion can be carried out by membrane filtration (Blasey and Jäger, 1991), settling devices (Thomson and Wilson, 1994), (dis)continuous centrifugation (Johnson et al., 1996), spinfilters (Himmelfarb et al., 1969) or two-step sequential sedimentation (Wen et al., 2000). However, all these methods present drawbacks limiting their use to short cultivation periods and/or low cell concentrations (Bibila and Robinson, 1995).

Another perfusion technique known as "sonoperfusion" or "acoustic perfusion" has also been proposed and is becoming a reference method, progressively superseding all other approaches developed so far (Zhang et al., 1998), since it has no moving part, is fast and efficient and it does not show clogging.

In this approach, the establishment of a standing (resonating) acoustic wave in the fluid results in the formation of velocity nodes or antinodes to which the biological products are forced to migrate and where they accumulate by the radiation force, depending on their compressibility and density.

One example of an acoustic cell filtration device is Applisense's BioSep acoustic biofilter (The Netherlands). This device requires three (peristaltic) pumps.

One of said peristaltic pumps sets the perfusion rate of the method and withdraws, following sonofiltration, the spent growth medium rid of cells from the bioreactor, so that it accumulates into a harvest reservoir. A second pump continuously recirculates the suspension of cells to and fro the acoustic biofilter at twice the perfusion rate, while a third pump brings fresh medium from a feed reservoir directly into the bioreactor.

However, continuous recirculation of the cell suspension to and from the acoustic biofilter constitutes a severe limitation of the acoustic perfusion technique.

Indeed, said recirculation, driven by the second pump, generates such severe mechanical forces (shearing forces) that fragile cells (insect cell lines such as Sf9, High Five, hybridomas,...) undergo a progressive decline in viability, while anchorage-dependent cells detach from their microsupport. After long cultivation periods, even shear-resistant mammalian cells (CHO, HeLa) show metabolic alterations so much so as to have their viability and specific productivity decreased. Because of the 2:1 ratio of the recirculation-to-perfusion rate, shear forces hinder cultivation at very high concentrations (higher than 50x10⁶ cells/ml), when perfusion rates reach values of three bioreactor culture volumes a day or higher.

### Aims of the invention

The present invention aims to provide a method and an apparatus for cultivating cells at high cell concentration in a bioreactor, in particular a sonoperfused bioreactor used for the production of cells or of biological products, which do not present the drawbacks of already existing perfusion techniques.

The present invention also aims to provide a method and an apparatus for cultivating cells at high cell concentrations in said bioreactor, wherein the viability of said cells is increased.

Another aim of the present invention is to provide a method and an apparatus with high productivity which require no additional costs as compared to the techniques of the state of the art.

### Summary of the invention

The present invention is related to an apparatus for obtaining and recovering biological products of interest from a cell culture (prokaryotic and/or eukaryotic cells, anchorage-dependent or not) in a bioreactor at a high cell concentration, comprising:
- a feed reservoir comprising fresh growth medium,
- a bioreactor containing the cell culture consisting of cells and appropriate growth medium,
- a biofilter for filtering the cell culture and retaining the cells, but not the growth medium,
- a harvest reservoir for collecting the spent growth medium out of the biofilter, and coming from the bioreactor,
- a first pump for transferring part of the cell culture from the bioreactor onto the biofilter and for transferring the filtered growth medium rid of cells from the biofilter into the harvest reservoir,
- a second pump for injecting fresh growth medium from the feed reservoir into the bioreactor and for recirculating cells out of the biofilter into the bioreactor, so as define a first cell circulation circuit,
- and a set of two flow controlling valves consisting of a first valve and second valve able to work alternately;
wherein the bioreactor, the first valve, the biofilter, the first pump and the harvest reservoir together define a first fluid circulation circuit;
and wherein the feed reservoir, the second pump, the biofilter, the second valve and the bioreactor together define a second fluid circulation circuit.

Preferably, said apparatus according to the present invention comprises a third fluid circulation circuit defined by the first pump the biofilter, the second valve and the bioreactor, the first pump being able to recirculate cells retained on the biofilter, from he biofilter into the bioreactor, following a second cell recirculation circuit.

Preferably, the second pump is an unidirectional pump, the first pump being either an unidirectional pump or a bidirectional pump.

Advantageously, the first valve and the second valve are placed between the bioreactor and the biofilter, the first pump being placed between the biofilter and the harvest reservoir.

The present invention is also related to a method for obtaining biological products from cells cultivated in a bioreactor at high cell concentrations, using the apparatus disclosed hereabove, said method comprising, according to a first preferred embodiment, the following steps:
- Step 1: the first valve is open and the first pump operates the transfer from the bioreactor of part of the cell culture contained therein onto the biofilter where sonoperfusion takes place, thereby fractionating the cell culture into two fractions, a first fraction corresponding to the cells which are trapped on the biofilter, while the second fraction corresponds to the growth medium and goes through the biofilter into the harvest reservoir;
- Step 2: the first valve is shut and the first pump is desactivated, while the second valve is open and the second pump operates both the injection of fresh growth medium from the feed reservoir into the bioreactor and the recirculation of the cells previously trapped on the biofilter from the biofilter into the bioreactor.

Preferably, according to a second preferred embodiment, the method further comprises an intermediate step, wherein the first valve is shut, the second valve is open and the first pump is reversed so that the first pump recirculates the cells previously trapped on the biofilter from the biofilter into the bioreactor.

Finally, the present invention is also related to the use of said apparatus or said method for the production of biological products of interest.

### Short description of the drawings

Fig. 1 represents the fluid circulation circuits with their different elements in the apparatus for the production of biological products using a bioreactor according to the state of the art.

Fig. 2 represents the fluid circulation circuits with their different elements in the apparatus for the production of biological products using a bioreactor according to the present invention.

Fig. 3A, 3B, 3C represent images obtained by microscopy of a selected area of the cytoplasm proteome of CHO-K1 cells grown in different conditions: at low cell concentration in batch mode (2,6x10⁶ cells/ml - figure 3C), at high cell concentration using the apparatus of the state of the art and disclosed on figure 1 (30 x10⁶ cells/ml-figure 3B), or at high cell concentration using the apparatus according to the invention disclosed on figure 2 (30 x10⁶ cells/ml - figure 3A).

### Detailed description of the state of the art

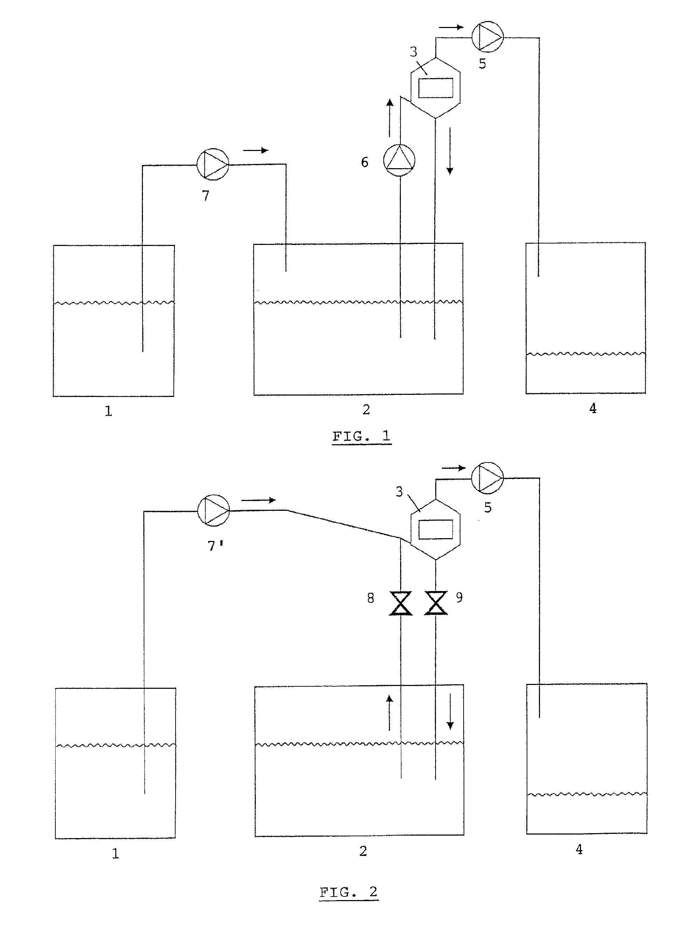

Figure 1 gives an example of an apparatus for producing biological products of interest from cells according to the state of the art. Said apparatus comprises:
- a feed reservoir 1 filled with a fresh growth medium kept at 4°C;
- a bioreactor 2 containing the cell culture (containing prokaryotic or eukaryotic cells and growth medium at least partially spent) ;
- a biofilter 3 which is an acoustic biofilter;
- a harvest reservoir 4, kept at 4°C, to collect the spent growth medium rid of cells after sonoperfusion.

The apparatus comprises also three pumps 5, 6, 7, which are preferably unidirectional pumps.

The method according to the state of the art comprises the following steps:
- the first pump 5 operates the transfer of the cell culture from the bioreactor 2 onto the acoustic biofilter 3 wherein sonoperfusion takes place, so that cells are separated from the spent growth medium, the cells being retained as a cell suspension onto the biofilter 3, while the spent growth medium is directed by the first pump 5 into the harvest reservoir 4.
- the second pump 6 operates the recirculation of the cell suspension to and from the acoustic biofilter 3, the cell suspension being redirected into the bioreactor 2,
- the third pump 7 brings transiently fresh growth medium containing nutrients from the feed reservoir 1 directly into the bioreactor, transiently restoring the volume of the culture at its pre-set value.

It should be noted that pump 5 sets the perfusion rate of the method and that pump 6 operates at twice the perfusion rate (determined by the cell concentration of the cell culture in the bioreactor 2), which triggers dramatic metabolic damages of the cells at high cell concentrations.

The spent growth medium in the harvest reservoir contains the biological products of interest, and will be treated in order to recover said biological products.

For performing this method three fluid circulation circuits are used:
- the bioreactor 2, the acoustic biofilter 3, the first pump 5 and the harvest reservoir 4 together define the first fluid circulation circuit;
- the feed reservoir 1, the third pump 7 and the bioreactor 2 together form the second fluid circulation circuit;
- the bioreactor 2, the second pump 6 and the acoustic biofilter 3 form the third fluid circulation circuit.

Another preferred embodiment of the invention is illustrated in figure 2. In this embodiment, the pump 6 is advantageously substituted by a set of two valves, a first valve 8 and a second valve 9 acting reciprocally (when valve 8 is open, valve 9 is shut and vice versa).

More precisely, the apparatus also comprises a bioreactor 2, a biofilter 3, a feed reservoir 1 and a harvest reservoir 4, as well as a first peristaltic pump 5 and a peristaltic pump 7' which will be called second pump hereafter.

During a first step, while pump 7' is not activated and the second valve 9 is shut, the first valve 8 is open, and following a first fluid circulation circuit, the first pump 5 transfers the cell culture from the bioreactor 2 into the biofilter 3, where it is filtered by acoustic filtration (also called sonoperfusion).

This acoustic filtration results in the separation of the cell culture into the cells and spent growth medium. More precisely, this filtration results into the agglomeration of the cells in the biofilter 3 in the form of a cell suspension, under the effect of the resonating standing acoustic wave, while the spent growth medium, now rid of cells ("sonoperfusate"), is transferred by the first pump 5 into the harvest reservoir 4. In this first step, the first fluid circulation circuit comprises thus the bioreactor 2, the first valve 8, the first pump 5, the biofilter 3 and the harvest reservoir 4.

The spent growth medium in the harvest reservoir contains the biological products of interest, and will be treated in order to recover said biological products.

During a second step, while the first valve 8 is shut and the first pump 5 is not activated, the second valve 9 is open and the second pump 7' operates the transfer of fresh growth medium from the feed reservoir 1 into the bioreactor 2, via the acoustic biofilter 3 and the second valve 9 so that cells previously agglomerated in said acoustic biofilter 3 are recirculated into the bioreactor 2. Said acoustic biofilter 3 gets rid of the agglomerated cells it contained by the action of a guzzling stream of fresh growth medium delivered by the peristaltic pump 7'. This second step is performed following the second fluid circulation circuit, which thus comprises the feed reservoir 1, the second pump 7', the biofilter 3, the second valve 9 and the bioreactor 2.

In this first preferred embodiment, the recirculation of cells is discontinuous. The first pump 5 and the second pump 7' are both unidirectional pumps.

Possibly, in a second preferred embodiment, the first pump 5 is a bi-directional pump instead of a unidirectional pump as in the first embodiment, the method further comprising an intermediate step, step 1', wherein the first valve 8 is shut, the second valve 9 is open and the first pump 5 operates the return of the cells (cell suspension) into the bioreactor 2.

In this second embodiment, there is thus a third fluid circulation circuit including the first pump 5, the acoustic biofilter 3, the second valve 9 and the bioreactor 2.

As in the first embodiment, the recirculation of cells is discontinuous, on the contrary to the methods according to the state of the art.

It should be noted that the rerouting into the bioreactor 2 of the cells (cell suspension) trapped on the acoustic biofilter 3 is performed in an optimized way, since at least one recirculation, but preferably two recalculations of the cells are ensured, the first recirculation occuring during step 2 by the second pump 7' (called "first cell recirculation circuit"), and advantageously the second recirculation occuring during step 1' by the first pump 5 working for a shortwhile in reverse (called "second cell recirculation circuit").

As emptying of the acoustic biofilter 3 (and therefore recirculation of the cells) is almost quantitative, improved sonoperfusion is obtained.

Moreover, in these two preferred embodiments, as cells are never continuously recirculated, on the contrary to the methods of the state of the art, cells are less traumatised.

Cellular uptake of glucose and production of lactate was measured and compared for cells grown in a bioreactor according to the method of the state of the art (conventional method) and cells grown in a bioreactor according to the method of the present invention, at different cell concentrations. Results are summarized in Table 1.

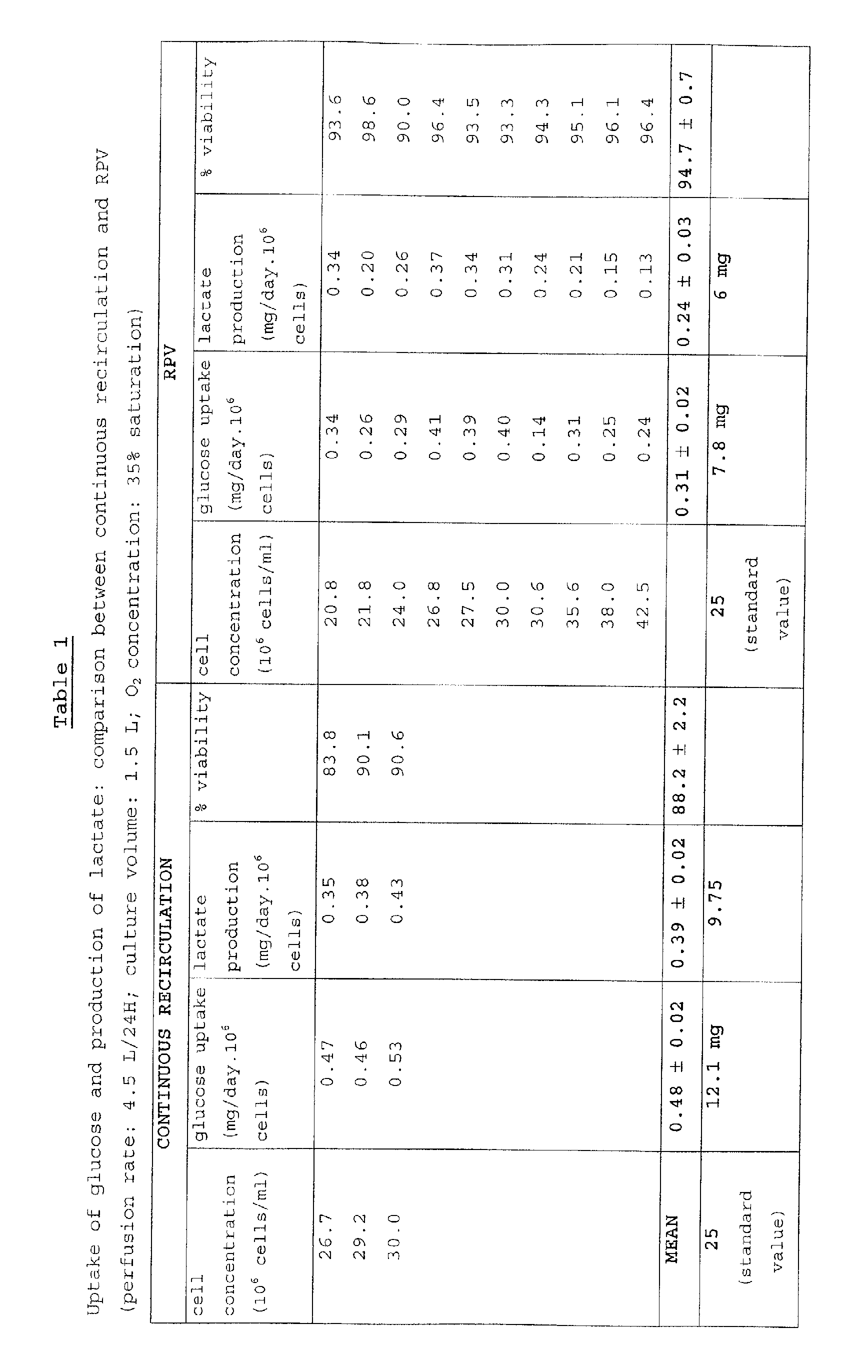

Table 1 shows that at high cell concentrations, the method according to the invention allows a 35.5% decrease of the glucose consumption and a comparable decrease in the lactate release (38.4%), as compared to the method according to the state of the art.

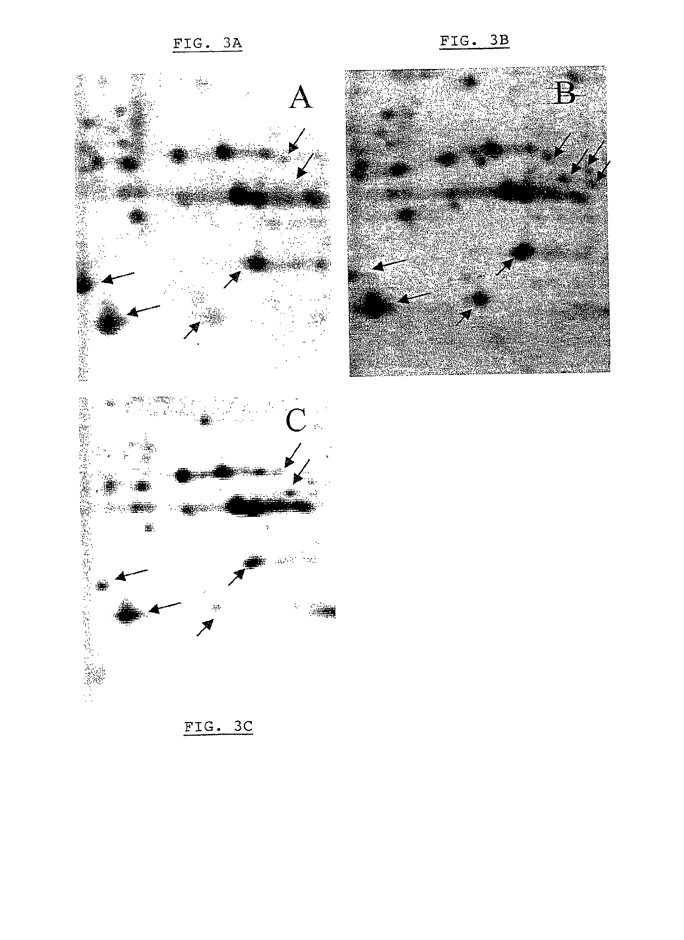

Moreover, as illustrated in figure 3, taking as reference a selected area of the cytoplasm proteome of cells grown at low cell concentration in batch mode (2,6x10⁶ cells/ml - figure 3C), the corresponding proteome of the same cells grown at high concentration (30x10⁶ cells/ml - figure 3A) according to the method of the present invention shows less changes as compared to the proteome of cells grown at high concentration (30x10⁶ cells/ml - figure 3B) according to the method of the state of the art. This indicates that the method according to the invention allows to grow cells at high concentrations with more limited metabolic damages than the method of the state of the art.

As a conclusion, the method according to the invention presents the advantage of preventing the shear forces acting on the cells which were previously caused by the working of pump 6 to recirculate cells after sonoperfusion in the method of the state of the art (figure 1). In particular, the problem of having a recirculation pump 6 working at twice the perfusion rate does no longer exist, so that the viability of the cells as well as the duration of the culture are increased.

Moreover, the performing of said efficient method does not require additional costs as compared to the method of the state of the art.

This method could therefore be advantageously used in biotechnology, especially for the production of biomolecules (bioproducts such as vaccines, monoclonal antibodies) of therapeutic - or industrial interest where productivity and security are of prime concern.

### REFERENCES

1. Bibila TA et al. (1995), Biotechnol. Prog. 11, 1-13.
2. Blasey HD et al. (1991), In: Sasaki R, Ikura K, eds. Animal cell culture production of biologicals, Dordrecht: Kluwer, p.61-73.
3. Himmelfarb P et al. (1969), Science 164, 555.
4. Johnsson M et al. (1996), Biotechnol. Prog. 12, 855-864.
5. Miller AOA (2001), Combining cell culture and process operation, Genet Eng. News 21, 29.
6. Thompson KJ et al. (1994), In: Spier RE, Griffiths JB, Berthold W. eds. Animal cell technology, Butterworth-Heinemann, p.227-229.
7. Wen Z-Y et al. (2000), J.Biotechnol.79, p.1-11.
8. Zhang J et al. (1998), Biotechnol. Bioeng 59, p.351-359.

## Claims

1. An apparatus for obtaining biological products from a cell culture in a bioreactor at high cell concentrations, comprising:
- a feed reservoir (1) comprising fresh growth medium,
- a bioreactor (2) containing the cell culture consisting of cells and growth medium,
- a biofilter (3) for filtering the cell culture and retaining the cells but not the growth medium,
- a harvest reservoir (4) for collecting the spent growth medium out of biofilter (3),
- a first pump (5) for transferring part of the cell culture from the bioreactor (2) onto the biofilter (3) and for transferring the filtered growth medium rid of cells from the biofilter (3) into the harvest reservoir (4)
- a second pump (7') for injecting fresh growth medium from the feed reservoir (1) into the bioreactor (2) and for recirculating cells out of the biofilter (3) into the bioreactor (2), so as define a first cell circulation circuit,
- and a set of two flow controlling valves consisting of a first valve (8) and second valve (9) able to work alternately;
wherein the bioreactor (2), the first valve (8), the biofilter (2), the first pump (5) and the harvest reservoir (4) together define a first fluid circulation circuit;
and wherein the feed reservoir (1), the second pump (7'), the biofilter (3), the second valve (9) and the bioreactor (2) together define a second fluid circulation circuit.

2. The apparatus according to claim 1, comprising a third fluid circulation circuit defined by the the first pump (5) the biofilter (3), the second valve (9) and the bioreactor (2), the first pump (5) being able to recirculate cells retained on the biofilter (3), from he biofilter (3) into the bioreactor (2), following a second cell recirculation circuit.

3. The apparatus according to claim 1 or 2, wherein the second pump (7') is an unidirectional pump and the first pump (5) is either an unidirectional pump or a bidirectional pump.

4. The apparatus according to any one of the preceding claims, wherein the first valve (8) and the second valve (9) are placed between the bioreactor (2) and the biofilter (3), the first pump (5) being placed between the biofilter (3) and the harvest reservoir (4).

5. A method for obtaining biological products from cells cultivated in a bioreactor at high cell concentrations, using the apparatus according to any one of the preceding claims, said method comprising the following steps:
- Step 1: the first valve (8) is open and the first pump (6) operates the transfer from the bioreactor (2) of part of the cell culture contained therein onto the biofilter (3) where sonoperfusion takes place, thereby fractionating the cell culture into two fractions, a first fraction corresponding to the cells which are trapped on the biofilter (3), while the second fraction corresponds to the growth medium and goes through the biofilter (3) into the harvest reservoir (4);
- Step 2: the first valve (8) is shut and the first pump (5) is desactivated, while the second valve (9) is open and the second pump (7') operates both the injection of fresh growth medium from the feed reservoir (1) into the bioreactor (2) and the recirculation of the cells previously trapped on the biofilter (3) from the biofilter (3) into the bioreactor (2).

6. The method according to claim 5, which further comprises an intermediate step, wherein the first valve (8) is shut, the second valve (9) is open and the first pump (5) is reversed so that the first pump (5) recirculates the cells previously trapped on the biofilter (3) from the biofilter (3) into the bioreactor (2).

7. Use of the apparatus according to any one of claims 1 to 4 or the method according to any one of claims 5 and 6 for the production of biological products.
